# EUROPEAN PATENT APPLICATION

(11) **EP 4 834 415 A1**
(43) Date of publication of application: **04.11.2026**
(21) Application number: 24913058.4
(22) Date of filing: 26.12.2024
(51) Int. Cl.: A61N 1/34

(54) **HIGH FREQUENCY TREATMENT DEVICE AND HIGH FREQUENCY TREATMENT METHOD**

(30) Priority: 29.12.2023 JP 2023223782
(71) Applicant: Kabushiki Kaisha Top, Adachi-ku, Tokyo 120-0035 (JP)
(72) Inventor: IKEUCHI, Atsushi, Tokyo 120-0035 (JP); MATSUMOTO, Takero, Tokyo 120-0035 (JP)
(74) Representative: Nederlandsch Octrooibureau
(86) International application number: PCT/JP2024/046286
(87) International publication number: WO 2025/143172

(57) **Abstract**

[PROBLEM TO BE SOLVED] Provided are a high-frequency treatment device and a high-frequency treatment method, which enable intermittent high-frequency current to flow more effectively to a treatment object.

A high-frequency treatment device is provided with an output unit for outputting high-frequency power, an electrode connected to the output unit and arranged on the treatment object, and a control unit for controlling the output unit so that the high-frequency power is intermittently output based on a preset output pattern, wherein the output pattern is set so that a plurality of output periods T1 for outputting the high-frequency power are set at different time lengths, and the output periods T1 are generated at different individual periods T5 for each of the plurality of output periods T1.

## Description

### [Field of the invention]

The present invention relates to a high-frequency (Radiofrequency) treatment apparatus and high-frequency treatment method for treating an object such as a human or an animal by passing a radiofrequency current therethrough.

### [Background of the invention]

Pulsed radiofrequency (PRF) has been known as a treatment method for chronic pain. In this pulsed radiofrequency method, a radiofrequency current is passed intermittently (in the form of pulses) through an electrode in a living body to be treated, and the generated electric field affects a nerve tissue or the like to reduce pain (For example, see Non-Patent Document 1.).

In general, the pulse high-frequency method is performed by applying a pulse wave of a high-frequency current (e.g., 480 kHz) to a living body at a constant frequency (e.g., 2 Hz) and a constant pulse width (e.g., 20 milliseconds) for several minutes. In other words, the conventional pulse high-frequency method is performed by applying a high-frequency current intermittently for several minutes at a constant output period (e.g., 20 milliseconds) and a constant output stop period (e.g., 480 milliseconds). In the pulse high-frequency method, the temperature around the electrode is kept below a predetermined threshold value (42-45°C.) during the treatment in order to prevent degeneration of nerve tissue.

### [Prior Art Document]

### [Non-Patent Document]

[Non-Patent Document 1] Yuko Nishiwaki and Sei Fukui, "Pulse high-frequency therapy (PRF) device," Medical Instrumentation, Japanese Society of Medical Devices, 2020, Vol. 90, No. 3, p. 290-295

### [Outline of Invention]

### [Problem to be solved by the invention]

However, in a conventional apparatus for performing a pulse high-frequency method, when a temperature measurement value exceeds a threshold value during treatment, the temperature around the electrode is kept below the threshold value by performing control such as reducing the output voltage of the high-frequency power or stopping the output of the high-frequency power.

Therefore, when the output voltage or the duty ratio (a value obtained by dividing the pulse width by the period) is set to a high value so as to increase the effect on nerve tissue or the like, the temperature around the electrode also tends to rise. Consequently, the output voltage may be unintentionally reduced, or the output may be stopped during treatment, regardless of the intention of the user (e.g., a physician). If such events occur frequently, a sufficient analgesic effect cannot be obtained.

The present invention has an object to provide a high-frequency treatment apparatus and a high-frequency treatment method, which enable intermittent high-frequency current to flow more effectively to a treatment object.

### [Means for solving problems]

The radiofrequency (High-frequency) treatment apparatus of the present invention includes an output unit for outputting high-frequency power, an electrode connected to the output unit and arranged on the treatment object, and a control unit for controlling the output unit so that the high-frequency power is intermittently output based on a preset output pattern, wherein the output pattern is set so that a plurality of output periods for outputting the high-frequency power are set at different time lengths, and the output periods occur at different individual periods for each of the plurality of output periods.

In the high-frequency treatment method for performing treatment by intermittently outputting high-frequency power to an electrode arranged in a treatment object in a preset output pattern, the output pattern is set such that a plurality of output periods for outputting the high-frequency power are set at different time lengths, and the output periods occur at different individual cycles for each of the plurality of output periods.

According to the high-frequency treatment apparatus and the high-frequency treatment method of the present invention, the duty ratio can be appropriately changed during treatment, that is, a period of high duty ratio and a period of low duty ratio can be appropriately mixed in the treatment period, so that the temperature rise around the electrode can be suppressed in the period of low duty ratio while the nerve tissue or the like is reliably affected in the period of high duty ratio.

Thus, the intervention of output control for lowering the temperature around the electrode can be eliminated, the treatment can be completed as intended by the user, and the analgesic effect can be obtained as intended by the user. In other words, the intermittent high-frequency current can more effectively flow to the treatment object.

In the high-frequency treatment apparatus of the present invention, the output pattern is preferably set so that the longer the time length of the output period is, the longer the time length of the individual cycle is set in the plurality of set output periods.

Thus, the frequency of occurrence of the output period having a long time length is reduced to suppress the temperature rise around the electrode, and the frequency of occurrence of the output period having a short time length is increased to secure the degree of influence on the nerve tissue or the like. Further, since the duty ratio can be changed so as to fluctuate irregularly during the treatment instead of uniformly, it is possible to suppress adaptation of the treatment object to the change in the duty ratio and to flow intermittent high-frequency current to the treatment object more effectively.

Further, in the high-frequency treatment apparatus of the present invention, it is preferable that the output pattern is set so that the time length of the output period is proportional to the time length of the individual cycle in a plurality of set output periods.

According to this arrangement, since the fluctuation of the change in the duty ratio during the treatment can be approximated to the 1/f fluctuation having high affinity with the living body, it is possible to reduce a sense of incongruity or discomfort felt by the treatment object during the treatment and to flow intermittent high-frequency current to the treatment object more effectively.

### Effect of the Invention

According to the high-frequency treatment apparatus and the high-frequency treatment method of the present invention, it is possible to achieve an excellent effect of allowing intermittent high-frequency current to flow to the treatment object more effectively.

### Brief Description of the Drawings

FIG. 1 is a schematic diagram showing an external appearance of a high-frequency treatment apparatus according to an embodiment of the present invention;
FIG. 2 is a block diagram showing a schematic of an internal configuration of a high-frequency treatment apparatus;
FIG. 3 is a schematic diagram showing a waveform of AC power output by an output unit in a pulsed high-frequency method;
FIG. 4 is a table showing an output pattern according to an embodiment of the present invention;
FIG. 5 is a time chart showing an output mode of high-frequency power according to an embodiment of the present invention;
FIG. 6 is a graph showing a time-series change in the cumulative value of an output period in an output pattern;
FIG. 7 is a graph showing a temperature measurement result of an embodiment.

### [Embodiments of the Invention]

Embodiments of the present invention will be described with reference to the accompanying drawings.

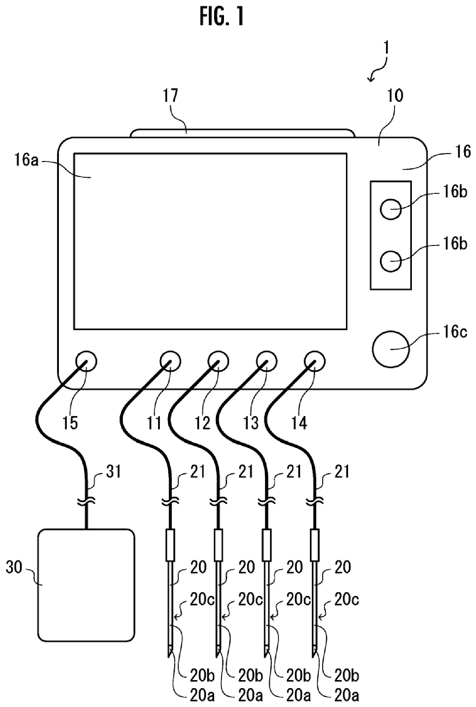

FIG. 1 is a schematic view showing an external appearance of a high-frequency treatment apparatus 1 according to an embodiment of the present invention. The high-frequency treatment apparatus 1 according to the present embodiment applies a high-frequency voltage to a living body such as a human being or an animal to be treated and flows a high-frequency current in the living body to perform a treatment for the purpose of reducing pain or the like.

The high-frequency treatment apparatus 1 is configured to perform not only a pulse high-frequency method in which an electric field formed in the living body acts on a nerve tissue or the like by intermittently flowing a high-frequency current in the living body, but also other treatments such as a high-frequency thermocoagulation method in which a nerve tissue is heated and denatured by the high-frequency current, and the like. Hereinafter, only the configuration and functions related to the pulse high-frequency method will be described.

As shown in FIG. 1, the high-frequency treatment apparatus 1 includes a main body 10, four electrodes 20 connected to the main body 10, and a counter electrode plate 30 connected to the main body 10.

The main body 10 accommodates or supports an internal configuration to be described later. On the front surface of the main body 10, four electrode connectors 11-14 to which the electrodes 20 are connected and a counter electrode plate connector 15 to which the counter electrode plate 30 is connected are provided below. An operation section 16 for receiving an operation by a user is also provided on the front of the main body 10. The operation section 16 comprises a touch panel display 16a for receiving an input operation such as various settings and displaying various information, a button 16b for receiving an operation for starting and ending a treatment, and a control knob 16c for adjusting an output voltage.

A handle 17 for carrying the main body 10 is provided on the upper part of the main body 10. Although not shown, a power supply connector and a power supply switch connected to a commercial AC power supply for receiving power are provided on the back of the main body 10.

The electrode 20 is inserted into an object to be treated in a high-frequency treatment to allow a high-frequency current to flow into the object. In this embodiment, the electrode 20 is formed into a needle tube that can be directly punctured into a human body or the like. Most of the electrode 20 is an insulating portion 20b coated with insulation except for a non-insulating portion 20a at the tip, and the high-frequency current is output from the non-insulating portion 20a. The electrode 20 also incorporates a thermocouple 20c for measuring the temperature around the electrode 20 during the treatment.

The electrode 20 is electrically connected to the main body 10 via an electrode cable 21 and an electrode connector 11-14. In this embodiment, a maximum of four electrodes 20 can be used simultaneously. The electrode 20 may have other shapes, such as a rod-shaped electrode inserted into a needle tube or a catheter, or a pad-shaped electrode arranged on the surface of a living body. The thermocouple 20c may be provided separately from the electrode 20.

The counter electrode plate 30 is a plate-shaped electrode which is attached to the surface of the object to be treated and is used for passing a high-frequency current to and from the electrode 20. The counter electrode plate 30 is electrically connected to the main body 10 via a counter electrode plate cable 31 and a counter electrode plate connector 15. Although the counter electrode plate 30 is formed in a rectangular flat plate shape in the present embodiment, the counter electrode plate 30 may have other shapes.

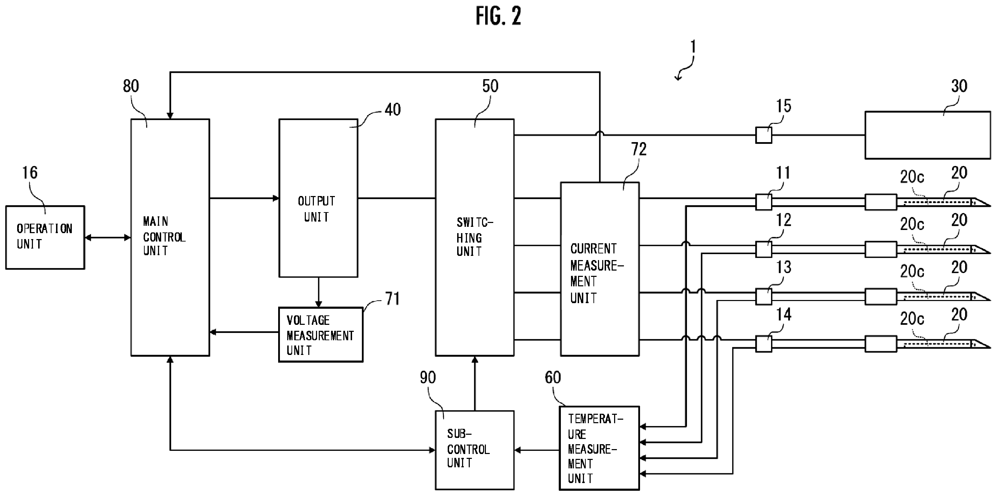

FIG. 2 is a block diagram schematically showing the internal configuration of the high-frequency treatment apparatus 1. As shown in FIG. 2, the high-frequency treatment apparatus 1 includes, as an internal configuration, an output unit 40, a switching unit 50, a temperature measurement unit 60, a voltage measurement unit 71, a current measurement unit 72, a main control unit 80, and a sub-control unit 90.

The output unit 40 generates and outputs high-frequency power of a preset frequency (For example, 470-490 kHz) and a voltage (For example, 18-80Vrms) based on a control signal from the main control unit 80 based on power supplied from a commercial AC power supply. The output unit 40 is connected to the electrode connectors 11-14 and the counter electrode plate connector 15 via the switching unit 50. Therefore, the electrode 20 is connected to the output unit 40 via the electrode connector 11-14 and the switching unit 50. Each of the output units 40 is composed of a known circuit having a transformer, so that the target to be treated is insulated from the commercial AC power supply.

The switching unit 50 operates under the control of the sub-control unit 90, and switches the connection state of the output unit 40 to the electrode connector 11-14 and the counter electrode plate connector 15 according to the treatment to be performed. The switching unit 50 of this embodiment is composed of a plurality of lead relays, but may be composed of a semiconductor switch or the like.

The temperature measurement unit 60 is constituted of a known circuit or the like, and generates a temperature measuring signal based on a signal received from the thermocouple 20c, and transmits the signal to the sub-control unit 90. In this embodiment, the temperature measurement unit 60 is connected to the thermocouple 20c in the electrode 20 via the electrode connectors 11-14, but a connector for the thermocouple 20c may be provided separately.

The voltage measurement unit 71 is constituted of a known circuit or the like, and measures the voltage of the high-frequency power output from the output unit 40. The voltage measurement unit 71 generates a voltage measuring signal based on the high-frequency voltage generated by the output unit 40, and transmits the signal to the main control unit 80. The current measurement unit 72 is constituted of a known circuit or the like, and individually measures the high-frequency current flowing through the four electrodes 20. The current measurement unit 72 generates a current measuring signal based on the high-frequency current flowing through the electrodes 20, and transmits the signal to the main control unit 80.

The main control unit 80 is provided with a known constitution such as a CPU, a ROM, and a RAM, and executes high-frequency processing by controlling each part of the high-frequency processing apparatus 1. The main control unit 80 starts and ends the high-frequency processing based on an input operation received by the operation unit 16 and information stored in a ROM or the like. During the processing, the main control unit 80 displays information such as elapsed time, measured voltage, measured current, and measured temperature on the touch panel display 16a.

Similar to the main control unit 80, the sub-control unit 90 has a known configuration such as a CPU, ROM, and RAM, and controls the switching unit 50 and the temperature measurement unit 60. In this embodiment, the sub-control unit 90 for controlling the switching unit 50 and the like provided on the processing target side relative to the output unit 40 is provided separately from the main control unit 80, so that insulation between the target and the commercial AC power supply can be made more complete.

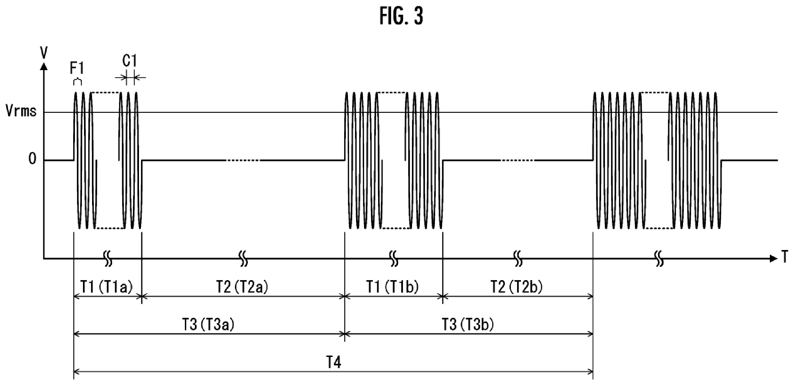

FIG. 3 is a schematic diagram showing the waveform of the AC power output by the output unit 40 in the pulsed high-frequency method, wherein the vertical axis represents the voltage V and the horizontal axis represents the time T. In the pulsed high-frequency method, the output unit 40 intermittently outputs high-frequency power of a predetermined frequency F1(For example, 480 kHz) under the control of the main control unit 80.

Specifically, as shown in FIG. 3, the output unit 40 outputs the high-frequency power during the output period T1a, then stops the output of the high-frequency power during the output stop period T2a, then outputs the high-frequency power during the output period T1b, then stops the output of the high-frequency power during the output stop period T2b, and outputs the high-frequency power in the manner... In other words, the output unit 40 outputs a pulse wave having a pulse width T1 and a period T3 longer than the high-frequency period C1.

The duty ratio in the period T3a is a value obtained by dividing the output period T1a by the period T3a, and the duty ratio in the period T3b is a value obtained by dividing the output period T1b by the period T3b. The average duty ratio in the period T4 is a value obtained by dividing the sum of the output period T1a and the output period T1b by the period T4.

Conventionally, in the pulsed high-frequency method, it has been common to output high-frequency power while keeping the output period T1 and the output stop period T2 constant, that is, to output a pulse wave having a constant pulse width T1 and a constant period T3. When the temperature measured around the electrode 20 exceeds a predetermined threshold (42-45°C., which is the upper limit of the temperature at which cells are not destroyed), output control such as reduction of the output voltage or stop of the output is performed.

Accordingly, the output voltage may be unintentionally reduced, or the output may be unintentionally stopped during treatment without the intention of the user of the apparatus, such as a physician. When this occurs frequently, a sufficient analgesic effect cannot be obtained.

Therefore, in the present embodiment, by outputting the high-frequency power while varying the output period T1 and the output stop period T2 as shown in FIG. 3 based on the preset output pattern, it is possible to suppress the temperature rise around the electrode 20 and to complete the treatment as intended by the user without performing the output control of the high-frequency power based on the temperature measurement value during the treatment.

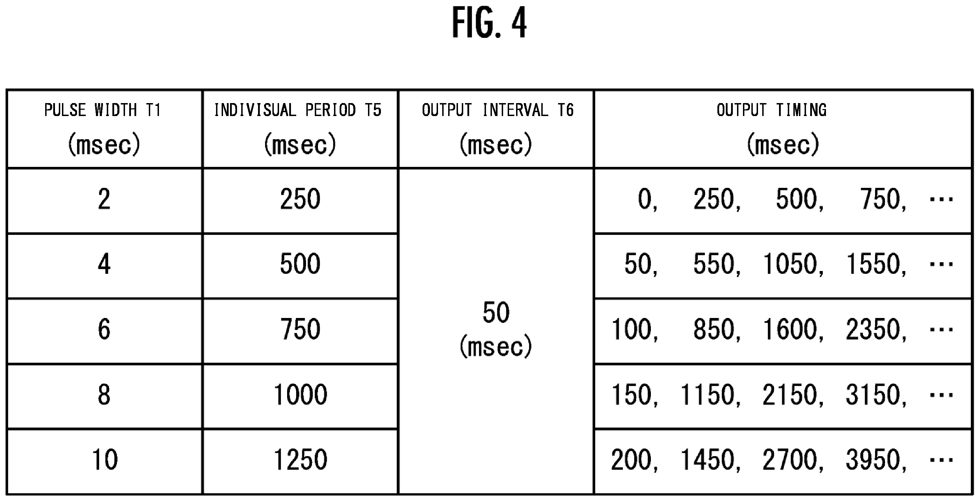

FIG. 4 is a table showing the output pattern of the present embodiment. In this output pattern, 5 kinds of time lengths of 2 milliseconds, 4 milliseconds, 6 milliseconds, 8 milliseconds, and 10 milliseconds are set as the pulse width T1, and they are set to be output in different individual periods T5. Specifically, a pulse having a pulse width T1 of 2 milliseconds is outputted at an individual period T5 of 250 milliseconds, a pulse having a pulse width T1 of 4 milliseconds is outputted at an individual period T5 of 500 milliseconds, a pulse having a pulse width T1 of 6 milliseconds is outputted at an individual period T5 of 750 milliseconds, a pulse having a pulse width T1 of 8 milliseconds is outputted at an individual period T5 of 1000 milliseconds, and a pulse having a pulse width T1 of 10 milliseconds is outputted at an individual period T5 of 1250 milliseconds.

That is, in the output pattern of this embodiment, the time length of the individual period T5 is set to be proportional to the time length of the pulse width T1, and the longer the time length of the output period T1 is, the longer the time length of the individual period T5 is in proportion to it.

In this output pattern, the output of the pulses of the pulse widths T1 is shifted by the output interval T6 so that the output timings of the pulses of the pulse widths T1 do not overlap. Specifically, in this output pattern, the output interval T6 is a value (50 milliseconds) obtained by dividing the shortest individual period T5 (250 milliseconds) by the number of pulse widths (5).

Therefore, the output timings of the pulses of the pulse width T1 in this output pattern are 0 ms (at the same time as the start), 250 ms, 500 ms, 750 ms, and ... from the output start for the pulse width T1 of 2 ms, 50 ms, 550 ms, 1050 ms, 1550 ms, and ... from the output start for the pulse width T1 of 4 ms, 100 ms, 850 ms, 1600 ms, 2350 ms, and ... from the output start for the pulse width T1 of 6 ms, 150 ms, 1150 ms, 2150 ms, 3150 ms, and ... from the output start for the pulse width T1 of 8 ms, and 200 ms, 1450 ms, 2700 ms, 3950 ms, and ... from the output start for the pulse width T1 of 10 ms.

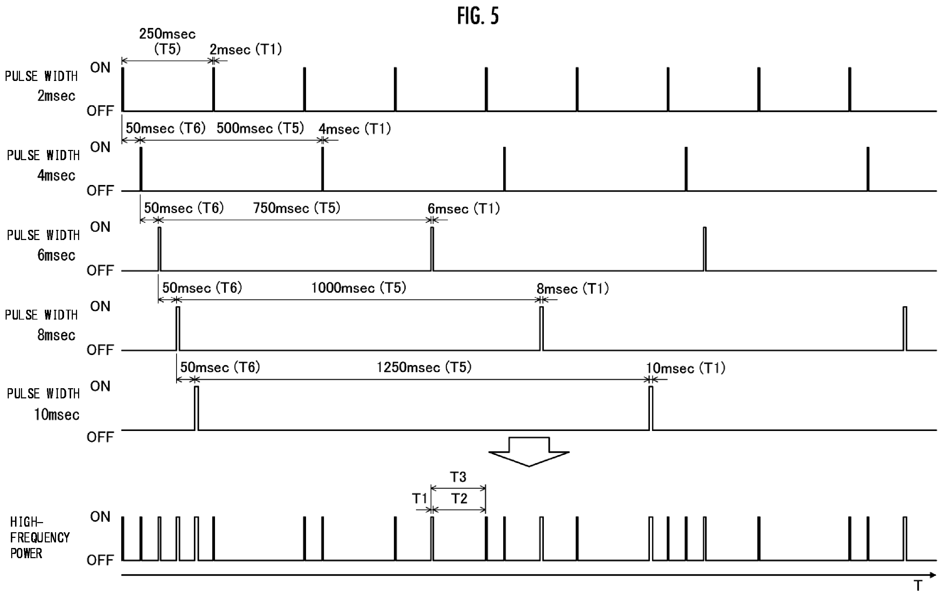

FIG. 5 is a time chart showing an output mode of the high-frequency power in this embodiment. The main control unit 80 controls on/off of the output of the high-frequency power from the output unit 40 based on the output pattern stored in the ROM, that is, executes output during the output period T1 and output stop during the output stop period T2.

As described above, pulses of each pulse width T1 are output at a constant individual period T5, and the timing at which the output starts is shifted by an output interval T6. Then, in this embodiment, by combining a plurality of regular pulse outputs of the constant pulse width T1 and the constant individual period T5, output of the high-frequency power is realized in a mode in which the output period (pulse width) T1 and the output stop period T2 vary irregularly as shown in the time chart at the bottom.

By varying the output period T1 and the output stop period T2 in this way, it is possible to suppress a rise in the temperature around the electrode 20 during a period of a low duty ratio while surely exerting an influence on the nervous tissue or the like during a period of a high duty ratio. Furthermore, by making the changes of the output period T1 and the output stop period T2 irregular, it is possible to suppress adaptation of the nerve tissue or the like to the electric field and to more effectively affect the nerve tissue or the like.

Further, in this embodiment, by setting the time length of the output period T1 to 10 milliseconds or less, it is possible to suppress excessive temperature rise while keeping the average duty ratio in one time of treatment equal to that of the conventional pulse high-frequency method (about 0.04). Further, by setting the time length of the individual period T5 to be longer as the time length of the output period T1 is longer, the frequency of occurrence of the output period T1 having a long time length is reduced to suppress temperature rise around the electrode 20, and the frequency of occurrence of the output period T1 having a short time length is increased to secure the degree of influence on the nerve tissue or the like.

In this embodiment, by fixing the individual period T5 of each output period T1, it is possible to easily calculate the average duty ratio in 1 treatment and to easily create an output pattern corresponding to the condition of the treatment object, the type of the nerve fiber to be treated, or the like.

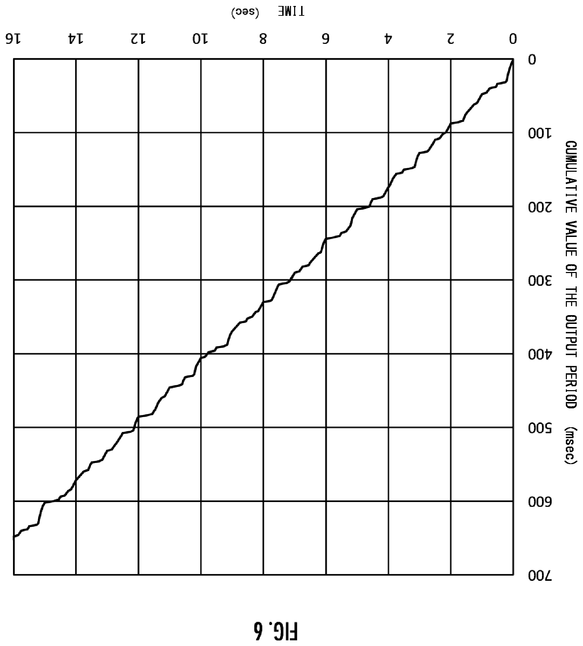

FIG. 6 is a graph showing a time-series change in the cumulative value of the output period T1 in the output pattern of this embodiment. In the graph of FIG. 6, the cumulative value of the output period T1 is plotted at the end of each output period T1. Therefore, the slope between the start point and the end point of any period in the graph of FIG. 6 is the average duty ratio in that period.

As shown in FIG. 6, in the output pattern of the present embodiment, the slope (i.e., duty ratio) of the graph of the cumulative value of the output period T1 shows a change that appears regular but is not regular, for example, a change such as a fluctuation of a flame of a candle or the like. In the output pattern of the present embodiment, since the least common multiple of the individual period T5 is 15000 milliseconds, the same change in the duty ratio is repeated every 15 seconds.

In the present embodiment, by setting the time length of the individual period T5 to a length corresponding to the time length of the output period T1, the duty ratio is changed so as to fluctuate irregularly during the treatment, and it is possible to more effectively suppress adaptation of the treatment target to the change in the duty ratio. Furthermore, by setting the time length of the output period T1 and the time length of the individual period T5 so as to be proportional (the individual frequency is inversely proportional), the fluctuation of the change in the duty ratio during the treatment is approximated to a 1/f fluctuation having a high affinity with the living body, and it is possible to reduce discomfort or discomfort felt by the treatment target during the treatment.

Although the present embodiment uses 5 kinds of time lengths of the output period T1 of 2-10 milliseconds, the length and the number of kinds of the time lengths of the output period T1 are not limited to this, and any value corresponding to the state of the treatment target or the like can be adopted. The same applies to the individual period T5 and the output interval T6.

The order of starting the output of the pulses of the respective time lengths is not particularly limited, and any order may be adopted. The time length of the individual period T5 is preferably set to a length corresponding to the output period T1, but other settings may be adopted depending on the condition of the treatment object or the like. It goes without saying that a plurality of kinds of output patterns may be used instead of using only one output pattern in one treatment.

The high-frequency treatment apparatus 1 may have only one of electrode connectors 11-14, and in this case, the switching unit 50 may be omitted. The high-frequency treatment apparatus 1 may have a plurality of counter electrode plate connectors 15. The high-frequency treatment apparatus 1 may have a plurality of output units 40 or a plurality of temperature measurement units 60 in accordance with the number of electrode connectors 11-14. The high-frequency treatment apparatus 1 may have no sub-control unit 90, and the main control unit 80 may control the switching unit 50 and the temperature measurement unit 60.

The high-frequency treatment apparatus 1 may be capable of executing the pulse high-frequency method in a fixed output period T1 and output stop period T2 set by the user, in addition to executing the pulse high-frequency method based on the output pattern stored in the ROM. The high-frequency processing apparatus 1 may be capable of executing the pulse high-frequency method based not only on the output pattern stored in the ROM but also on the output pattern stored in the flash memory or the like connected to the main control unit 80 or the output pattern acquired by communication with an external device.

Although the above-described embodiments of the present invention have been described, the high-frequency processing apparatus and the high-frequency processing method of the present invention are not limited to the above-described embodiments, and it is needless to say that various modifications may be made within a range not departing from the gist of the present invention.

Furthermore, the operations and effects shown in the above-described embodiments are only those which are most suitable and are derived from the present invention, and the operations and effects of the present invention are not limited thereto.

### [Example]

Next, an example of the present invention will be described.

An electrode and a counter electrode plate were arranged on a pork, and a high-frequency current was passed at a voltage of 70 V based on an output pattern in which the average duty ratio was set to 0.015 by 10 different output periods (pulse widths), and the temperature around the electrode was measured by a thermocouple built in the electrode. The measurement time was 540 seconds (9 minutes), and the sampling frequency of the temperature measurement was 1 Hz. The initial temperature of the pork was set at room temperature, and the impedance value was adjusted to 200 Ω by adjusting the contact area between the counter electrode plate and the pork.

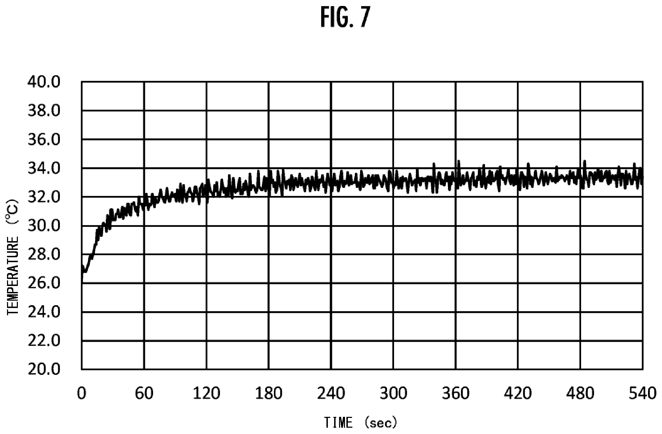

FIG. 7 is a graph showing the temperature measurement results of the embodiment. As shown in FIG. 7, according to the output pattern of the embodiment, it was confirmed that the temperature around the electrode rose by about 6-7°C. after the start of the output, and was maintained at a substantially constant value while the output was continued thereafter. In other words, the effectiveness of the present invention was confirmed.

### [Explanation of Signs]

1. High-Frequency Treatment Apparatus
20 Electrodes
40 Output unit
80 Main control unit
T1 output period
T5 individual period

## Claims

1. The high-frequency treatment apparatus includes:
an output unit for outputting high-frequency power;
an electrode connected to the output unit and arranged in a treatment object;
and a control unit for controlling the output unit so that the high-frequency power is intermittently output based on a preset output pattern,
wherein the output pattern is set so that a plurality of output periods for outputting the high-frequency power are set at different time lengths and the output periods occur at different individual cycles for each of the plurality of output periods.

2. The high-frequency treatment apparatus according to claim 1, wherein
the output pattern is set so that the longer the time length of the output period is, the longer the time length of the individual period is.

3. The high-frequency treatment apparatus according to claim 1, wherein
the output pattern is set so that the time length of the output period is proportional to the time length of the individual period in the plurality of set output periods.

4. A high-frequency treatment method for performing treatment by intermittently outputting high-frequency power in a preset output pattern to an electrode arranged to be treated, wherein
the output pattern is set so that a plurality of output periods for outputting the high-frequency power are set at different time lengths, and the output periods occur at different individual periods for each of the plurality of output periods.
